# FASCICULE DE BREVET EUROPEEN

(11) **EP 1 616 528 B1**
(45) Date de publication et mention de la délivrance du brevet: **20.06.2012**
(21) Numéro de dépôt: 05356120.5
(22) Date de dépôt: 13.07.2005
(51) Int. Cl.: A61B 17/80

(54) **Implant de fixation pour l'ostéosynthèse de fragments d'un premier métatarsien après fracture ou ostéotomie dans sa partie proximale**
Fixier-Implantat zur Osteosysnthese von Fragmenten des ersten metatarsalen Knochens nach Bruch oder Osteotomie in seinem proximalen Teil
Fixation implant for osteosynthesis of fragments of the first metatarsal bone after fracture or osteotomy in its proximal part

(30) Priorité: 15.07.2004 FR 0407891
(43) Date de publication de la demande: 18.01.2006
(73) Titulaire: Newdeal, 69800 Saint-Priest (FR)
(72) Inventeur: Toullec, Eric, René, Marie, 33200 Bordeaux (FR); Alain, Jérome, 8700 Limoges (FR); Camelot, Christophe, Jean-Michel, Nicolas, 94300 Vincennes (FR); Graff, Wilfrid, Olivier, 75020 Paris (FR)
(74) Mandataire: Martin, Didier Roland Valéry

(56) Documents cités:
- EP-A- 1 452 145
- US-A1- 2004 102 777
- US-B1- 6 358 250
- US-B1- 6 508 819

## Description

La présente invention se rapporte au domaine technique général des implants de fixation destinés à assurer l'ostéosynthèse de fragments d'os, et notamment de deux fragments d'un os fracturé ou sectionné par ostéotomie.

La présente invention se rapporte plus particulièrement au domaine technique des dispositifs destinés au traitement de certaines pathologies de l'avant-pied telles que des fractures ou encore des déformations du gros orteil, notamment connues sous l'appellation scientifique « *hallux valgus* ».

La présente invention concerne un implant de fixation pour l'ostéosynthèse de fragments du premier métatarsien dans sa partie proximale, située vers le tarse, comprenant au moins un élément de fixation, destiné à être fixé, par l'intermédiaire d'une face de fixation, sur ou contre la surface externe du premier métatarsien, par exemple et essentiellement de part et d'autre de la zone de séparation entre les fragments d'os, en vue d'assurer leur solidarisation.

Il existe plusieurs techniques et plusieurs dispositifs associés pour corriger les déformations de type *« hallux valgus* ».

*L'« hallux valgus »* est une déformation de l'avant-pied qui s'observe par une déviation latérale exagérée du gros orteil. Une fois la déformation initiée, elle s'accentue généralement avec l'augmentation de l'angle métatarsophalangien. Cette déformation est souvent accompagnée d'une tuméfaction douloureuse communément dénommée « *oignon* ».

De nombreuses méthodes chirurgicales ont été proposées pour corriger les déformations du type *« hallux valgus* ». On distingue aujourd'hui deux catégories principales d'opérations, à savoir d'une part les ostéotomies dites distales qui consistent à sectionner le premier métatarsien au niveau de sa partie distale (ou tête du métatarsien) située vers la phalange, et les ostéotomies dites proximales qui consistent à sectionner le premier métatarsien dans sa partie proximale (ou base du métatarsien) située vers le tarse.

La première catégorie d'opérations donne généralement de bons résultats dans le cas de déviations angulaires faibles entre le premier et le deuxième métatarsiens. En revanche, cette catégorie d'intervention s'avère beaucoup moins satisfaisante dans le cas de fortes déviations angulaires et notamment lorsque l'angle entre le premier et le deuxième métatarsiens dépasse 10° ou 15°. Dans ce dernier cas, la seconde catégorie d'opérations est préférée. L'ostéotomie proximale consiste ainsi à sectionner, par exemple par abord latéral, le premier métatarsien en au moins deux fragments d'os, dans sa partie proximale située vers le tarse. On effectue ensuite un ré-alignement des fragments d'os de manière à corriger la déviation angulaire. L'ostéosynthèse des fragments d'os est ensuite réalisée à l'aide d'un système de fixation par exemple formé par des vis ou encore une plaque positionnée sur la partie dorsale ou latérale du premier métatarsien.

Les systèmes de fixations connus présentent cependant plusieurs inconvénients non négligeables, tant sur le plan de leur mise en place difficile que sur le plan de leur tenue mécanique et de leur capacité à maintenir efficacement les fragments d'os ensemble.

En effet, les systèmes de fixation à vis peuvent, en cas d'une compression trop forte, rendre l'os friable, ce qui peut provoquer la désolidarisation des fragments d'os.

Les systèmes à plaque donnent, de ce point de vue, de meilleurs résultats mais peuvent également poser des problèmes, en particulier au cours de la phase de consolidation de l'os. En effet, pendant cette période, l'os peut être soumis à des sollicitations mécaniques, notamment lors de la marche, qui sont susceptibles de provoquer la séparation des fragments d'os.

Dans le cas d'une opération d'« *hallux valgus* », il est généralement souhaitable que le patient retrouve le plus rapidement possible l'usage de son pied. Le pied opéré est donc remis rapidement en appui sur le sol au cours de la phase de consolidation, ce qui peut provoquer, lors de la marche et sous l'action du poids du corps du patient, un couple tendant à contraindre très fortement le système de fixation et à séparer les fragments d'os. Ce phénomène est généralement accentué dans le cas de plaques de fixation disposées sur la surface dorsale ou latérale de la partie proximale du premier métatarsien. En effet, les plaques de fixation dorsales travaillent en flexion et peuvent difficilement s'opposer au phénomène d'ouverture de l'os du côté plantaire, alors que l'os à justement tendance à s'ouvrir davantage du côté plantaire lors de la marche.

En outre, il arrive que les plaques de fixation soient mal positionnées, notamment en raison de leur mise en place souvent difficile. Ce mauvais positionnement est non seulement susceptible de nuire à la stabilité de l'implant mais peut également, sous l'effet de sollicitations mécaniques répétées, conduire à un relâchement progressif de l'implant, voire à son arrachement.

Le positionnement de l'implant de fixation s'avère donc être un paramètre fondamental pour que l'implant soit peu sensible aux sollicitations mécaniques extérieures, notamment liées à la marche, et assure une tenue mécanique optimale des fragments d'os ensemble.

Les documents US 6,358,250 B1 et US 6,508,819 B1 divulguent des plaques pour le poignet.

Le document EP 1 452 145 A1 appartient à l'état de la technique selon l'article 54(3) CBE et divulgue une plaque pour la première phalange d'un pied.

Les objets assignés à l'invention visent par conséquent à proposer un nouvel implant de fixation ne présentant pas les inconvénients énumérés précédemment et qui permette de réparer les fractures ou de corriger toute déformation, notamment les déformations de type *« hallux valgus »* les plus sévères, tout en autorisant une reprise rapide des activités physiques quotidiennes du patient, telles que la marche.

Un autre objet de l'invention vise à proposer un nouvel implant de fixation dont la résistance aux sollicitations mécaniques est supérieure à celle des dispositifs antérieurs, notamment dans la phase post-opératoire immédiate.

Un autre objet de l'invention vise à proposer un nouvel implant dont la stabilité n'est pas obérée par des contraintes répétées, évitant ainsi la séparation des fragments d'os lors de la consolidation.

Un autre objet de l'invention vise à proposer un nouvel implant de fixation dont la position est verrouillée, de telle sorte que l'implant ne puisse pas glisser lorsqu'il est sous contrainte, et notamment lors d'activités physiques telles que la marche.

Un autre objet de l'invention vise à proposer un nouvel implant de fixation qui soit particulièrement facile à mettre en place et à positionner de façon optimale sur la surface plantaire proximale du premier métatarsien.

Un autre objet de l'invention vise à proposer un nouvel implant de fixation qui permette une pose en aveugle.

Un autre objet de l'invention vise à proposer un nouvel implant de fixation dont l'encombrement est réduit, notamment vis-à-vis des tissus mous situés à proximité.

Un autre objet de l'invention vise à proposer un nouvel implant de fixation qui permette d'effectuer une compression optimale des fragments d'os et ce sans les fragiliser.

Un autre objet de l'invention vise à proposer un nouvel implant de fixation atraumatique.

Les objets assignés à l'invention sont atteints à l'aide d'un implant de fixation selon la revendication 1 pour l'ostéosynthèse de fragments du premier métatarsien dans sa partie proximale, située vers le tarse, comprenant au moins un élément de fixation, destiné à être fixé, par l'intermédiaire d'une face de fixation, sur ou contre la surface externe du premier métatarsien, en vue d'assurer la solidarisation des fragments d'os, ladite face de fixation comportant au moins une portion de surface anatomique de forme sensiblement conjuguée ou complémentaire à celle de la surface plantaire de la partie proximale du premier métatarsien.

D'autres objets et avantages de l'invention apparaîtront plus en détails à la lecture de la description qui suit, et à l'aide des dessins annexés, fournis à titre purement illustratif et non limitatif, dans lesquels :
- La figure 1 illustre, selon une vue de dessous, un squelette schématisé d'un pied gauche humain, sur lequel on a réalisé une ostéotomie de la partie proximale du premier métatarsien.
- La figure 2 illustre, selon une vue de dessous, le squelette d'un avant-pied humain présentant une déformation de type *« hallux valgus »* sur le premier métatarsien, avant opération.
- La figure 3 illustre, selon une vue de côté en élévation, le schéma d'un squelette d'un pied droit humain, sur lequel est monté un implant de fixation conforme à l'invention.
- Les figures 4 et 5 illustrent, selon une vue de côté, un élément de fixation conforme à l'invention.
- Les figures 6a et 6b illustrent, selon une vue de dessus, des éléments de fixation gauche et droit conçus spécifiquement pour un pied gauche et un pied droit.
- La figure 7 illustre, selon une vue de dessus, un mode de réalisation préférentiel de l'élément de fixation conforme à l'invention.

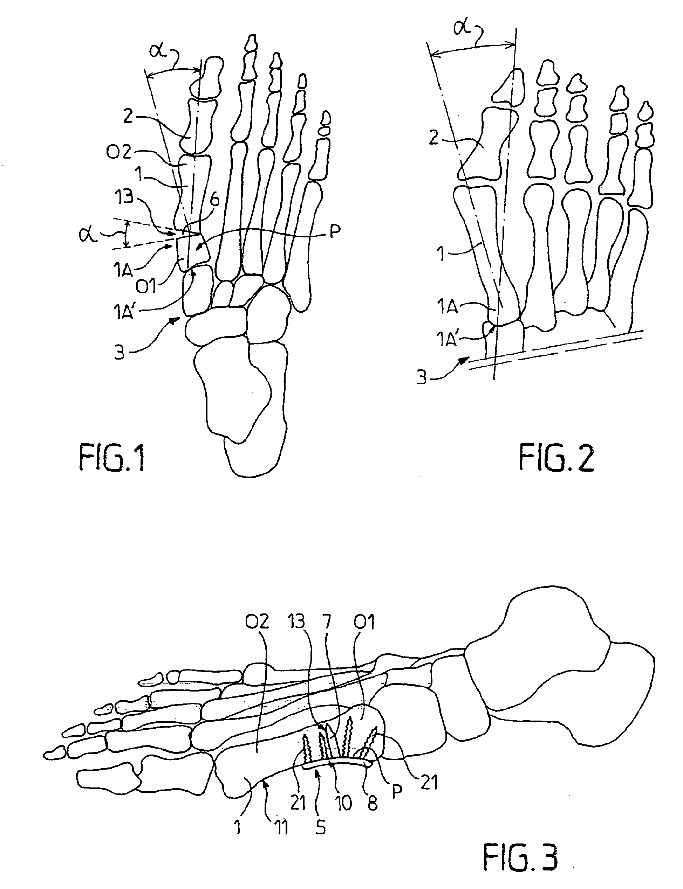

La figure 1 illustre le squelette d'un pied humain, sur lequel on distingue le premier métatarsien 1 interposé entre la première phalange 2 du gros orteil et le tarse 3. Tel que cela est illustré sur la figure 1, le premier métatarsien 1 a été sectionné, par abord latéral, dans sa partie proximale 1A située vers le tarse 3, pour corriger par exemple une déformation de type *« hallux valgus »* telle que celle illustrée sur la figure 2, correspondant à une déviation angulaire α anormale du premier métatarsien.

Dans la suite de la description, on considère que le premier métatarsien a subi une ostéotomie dans sa partie proximale 1A, le séparant par exemple en deux fragments d'os 01, 02. Dans ce cas, l'implant de fixation conforme à l'invention est destiné à assurer l'ostéosynthèse des deux fragments d'os 01, 02 suite à l'ostéotomie réalisée. L'implant de fixation conforme à l'invention n'est toutefois pas limité à ce type d'application et pourrait également être utilisé, sans sortir du cadre de l'invention, pour réparer des fractures de la partie proximale du premier métatarsien.

La figure 3 illustre un implant de fixation 5 fixé sur le premier métatarsien 1, en vue d'assurer l'ostéosynthèse des fragments d'os 01, 02, à la suite de l'ostéotomie illustrée sur la figure 1. En vue de combler l'espace de séparation 6 entre les deux fragments d'os 01, 02, un coin 7 (ou une cale) est disposé entre les deux fragments d'os O1, O2.

Au sens de l'invention, les fragments d'os 01, 02 ne sont pas nécessairement disjoints, c'est-à-dire que le premier métatarsien 1 n'est pas nécessairement sectionné ou fracturé sur tout son diamètre, mais qu'il peut subsister une zone de rattachement entre les fragments d'os 01, 02. L'implant de fixation 5 est ainsi destiné à assurer l'ostéosynthèse d'au moins deux fragments d'os 01, 02, mais éventuellement d'un nombre plus important de fragment d'os.

Selon l'invention, l'implant de fixation 5 comporte au moins un élément de fixation 8 préférentiellement formé par une plaque 9. L'élément de fixation 8, précisément la plaque 9 comporte une face de fixation 10 par l'intermédiaire de laquelle l'élément de fixation 8 est destiné à être fixé sur ou contre la surface externe 11 osseuse du premier métatarsien 1. La face de fixation 10 forme ainsi la face dite interne de l'élément de fixation 8. L'élément de fixation 8 comporte en outre, avantageusement, une face externe 12 sensiblement opposée à la face de fixation 10.

Les fragments d'os 01, 02 sont séparés au niveau d'une zone de séparation 13 de part et d'autre de laquelle l'élément de fixation 8 est destiné à être fixé en vue d'assurer la solidarisation et l'ostéosynthèse des fragments d'os O1, O2.

Selon une caractéristique essentielle de l'invention, la face de fixation 10 comporte au moins une portion de surface anatomique 14 de forme sensiblement conjuguée ou complémentaire à celle de la surface plantaire P de la partie proximale 1A du premier métatarsien 1.

De façon particulièrement avantageuse, la portion de surface anatomique 14 est conformée, c'est-à-dire spécifiquement conçue et dimensionnée, de manière à venir sensiblement épouser la surface plantaire P de la partie proximale 1A du premier métatarsien 1.

L'expression « *surface plantaire »* fait référence à la surface osseuse située sous la partie proximale 1A du premier métatarsien 1, et se différencie donc des surfaces osseuses dorsales et latérales du premier métatarsien.

En épousant anatomiquement la surface plantaire P proximale du premier métatarsien 1, et en venant en appui intime contre la surface osseuse, l'élément de fixation 8 offre une résistance améliorée aux sollicitations mécaniques, notamment liées à la marche. La conformation géométrique de l'élément de fixation 8 permet également un meilleur positionnement ainsi qu'un meilleur appui de l'implant sur les fragments d'os à solidariser, ce qui permet d'obtenir de meilleurs résultats sur le plan clinique.

En outre, la forme particulière de l'élément de fixation 8 lui permet d'être mis en place très rapidement, et en aveugle, puisqu'il vient naturellement se positionner à l'endroit voulu. L'élément de fixation 8 est ainsi préformé lors de sa fabrication, c'est-à-dire très en amont de l'intervention chirurgicale, conformément à l'aspect de la surface plantaire P proximale du premier métatarsien 1. La présente invention permet donc d'anticiper le positionnement de l'implant de fixation 5 et de rendre ce dernier immédiatement opérationnel et fonctionnel.

De façon particulièrement avantageuse, la portion de surface anatomique 14 de l'élément de fixation 8 est sensiblement convexe de manière à s'adapter à la concavité de la surface plantaire P proximale du premier métatarsien 1. L'élément de fixation 8 suit donc avantageusement la courbure de la surface plantaire P proximale du premier métatarsien 1, ce qui lui confère une très grande stabilité mécanique.

La portion de la surface anatomique 14 comprend une première surface dite surface proximale 15 située vers l'extrémité proximale 1A' du premier métatarsien 1.

La portion de surface anatomique 14 comprend au moins une surface distale 16, située vers l'extrémité de l'élément de fixation 8 opposée à la surface proximale 15, et une surface intermédiaire 17, située entre la surface proximale 15 et la surface distale 16, de telle manière que les surfaces proximale 15, intermédiaire 17 et distale 16 soient sensiblement inclinées les unes par rapport aux autres.

La position relative et l'orientation de ces surfaces sont primordiales puisqu'elles conditionnent le positionnement et l'orientation de l'implant de fixation 5. Ainsi, une mauvaise configuration spatiale de ces surfaces aurait un effet négatif sur la stabilité de l'implant et sa capacité à résister aux sollicitations mécaniques.

De façon préférentielle, les surfaces proximale 15, intermédiaire 17 et distale 16 sont sensiblement planes.

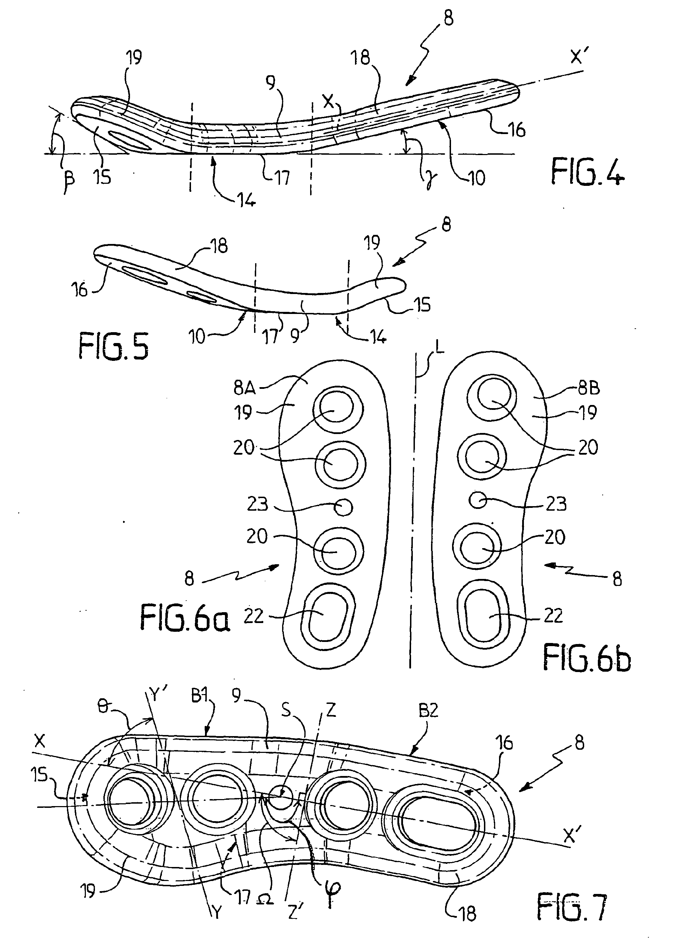

Selon une caractéristique particulièrement avantageuse de l'invention, les surfaces proximale et intermédiaire 15, 17 sont inclinées l'une par rapport à l'autre d'un angle de cintrage proximal ß compris entre 5° et 30° et préférentiellement de l'ordre de 25° (figure 4). Les surfaces proximale 15 et intermédiaire 17 se rejoignent ainsi au niveau d'un axe de cintrage proximal Y-Y' qui coïncide, à titre illustratif, avec un axe de pliage fictif de l'élément de fixation 8 de part et d'autre duquel ont inclinées les surfaces proximale et intermédiaire 15, 17.

Avantageusement les surfaces distale 16 et intermédiaire 17 sont inclinées l'une par rapport à l'autre d'un angle de cintrage distal γ compris entre 5° et 25° et préférentiellement de l'ordre de 15°. Les surfaces distale 16 et intermédiaire 17 se rejoignent avantageusement autour d'un axe de cintrage distal Z-Z' coïncidant sensiblement avec un axe de pliage fictif de l'élément de fixation 8 de part et d'autre duquel sont inclinées les surfaces distale 16 et intermédiaire 17.

L'élément de fixation 8 s'étend, de façon grossière, longitudinalement suivant un axe longitudinal X-X'. Cet axe longitudinal X-X' est sensiblement confondu avec l'axe d'extension longitudinale de la surface distale 16 ou du tronçon distal 18 de l'élément de fixation 8.

L'axe de cintrage proximal Y-Y' est avantageusement incliné par rapport à l'axe longitudinal X-X' d'un angle θ compris entre 60° et 80° et préférentiellement de l'ordre de 70°. L'axe de cintrage distal Z-Z' est quant à lui incliné, par rapport à l'axe longitudinal X-X' d'un angle Ω compris entre 80° et 100° et préférentiellement de l'ordre de 90°.

Avantageusement, l'élément de fixation 8 comporte, situé à l'opposé du tronçon distal 18, un tronçon proximal 19 en forme de spatule, sensiblement plus large que le reste de l'élément de fixation 8, et notamment plus large que le tronçon distal 18. Grâce à cette forme de spatule, le contact de l'élément de fixation 8 avec la surface osseuse est sensiblement amélioré, ce qui permet de mieux répartir les contraintes mécaniques exercées sur cette partie de l'implant de fixation 5. Ce dernier peut alors venir en appui intime et stable sur la surface osseuse du premier métatarsien 1, absorbant ainsi avec une grande efficacité l'ensemble des sollicitations mécaniques exercées sur les fragments d'os 01, 02.

Le tronçon proximal 19 en forme de spatule constitue également un moyen de reconnaissance tactile ou visuel d'un élément de fixation gauche 8A, destiné à être mis en place sur le premier métatarsien 1 du pied gauche par rapport à un élément de fixation droit 8B destiné à être mis en place sur le premier métatarsien du pied droit. Tel que cela est illustré sur les figures 6A et 6B, les éléments de fixation droit et gauche 8A, 8B sont symétriques par rapport au plan sagittal L, c'est-à-dire par rapport au plan vertical passant à travers le corps du côté antérieur vers le côté postérieur et divisant ainsi le corps en une moitié droite et une moitié gauche.

Tel que cela est illustré sur la figure 7, l'élément de fixation 8, comprend avantageusement au moins deux branches, à savoir une branche proximale B1 et une branche distale B2 disposées sensiblement en V. Ainsi, si l'on définit un plan sensiblement tangent à la surface distale 16 de l'élément de fixation 8, les branches B1, B2 s'étendent, en projection sur ce plan tangent, de manière à former un V avec un grand angle d'ouverture. L'angle ϕ au sommet S du V est avantageusement compris entre 160° et 175° et préférentiellement de l'ordre de 170°.

Afin de minimiser l'encombrement de l'implant de fixation 5, et notamment de limiter le caractère invasif et agressif de cet implant vis-à-vis des tissus mous environnant les fragments d'os, la face externe 12 de la plaque 9 s'étend préférentiellement de façon sensiblement parallèle à la face de fixation 10. Ainsi, l'intégralité de l'élément de fixation 8 vient épouser la forme du premier métatarsien 1 dans sa zone proximale 1A, et forme donc un implant de fixation 5 anatomique reproduisant les caractéristiques anatomiques du premier métatarsien 1, en vue de rendre l'implant invisible des tissus mous. En vue d'améliorer encore d'avantage le caractère atraumatique de l'implant de fixation 5, la face externe 12 est préférentiellement lisse et comporte des bords arrondis ou émoussés.

Au contraire, la face de fixation 10 présente préférentiellement un aspect rugueux, obtenu par exemple par sablage, afin de faciliter l'accrochage et l'adhérence de la face de fixation 10 sur la surface osseuse du premier métatarsien 1 et d'empêcher cette dernière de glisser ou de bouger vis-à-vis de l'os, une fois l'élément de fixation 8 mis en place dans sa position fonctionnelle.

La fixation de l'implant de fixation 5 sur la surface osseuse du premier métatarsien 1 constitue une étape cruciale de l'intervention chirurgicale dans la mesure où un mauvais positionnement de l'implant, de même qu'un serrage non uniforme de l'implant sur l'os peuvent conduire à la désolidarisation progressive des fragments d'os 01, 02. Dès lors, pour parfaire la fixation de l'implant de fixation 5 sur le premier métatarsien 1, l'élément de fixation 8 comporte avantageusement une pluralité de logements 20 débouchants répartis à intervalles réguliers sur la longueur de l'élément de fixation 8 et adaptés pour recevoir des éléments d'ancrage 21, du genre vis d'ancrage.

Afin de verrouiller la position de l'élément de fixation 8 sur le premier métatarsien 1, les éléments d'ancrage 21 comportent préférentiellement au moins une vis et une contre-vis, montées en association. A cet effet, les logements 20 débouchant comportent préférentiellement un filetage interne permettant le vissage de la contre-vis jusqu'à ce que cette dernière vienne en appui contre la tête de vis.

De tels systèmes de fixation, vis-contre-vis peuvent par exemple être basés sur le concept Surfix®, bien connu de l'homme du métier et qui ne fait donc pas l'objet d'une description détaillée.

De façon encore plus préférentielle, l'élément de fixation 8 comporte une ouverture oblongue 22 débouchante, ménagée préférentiellement à travers le tronçon distal 18 de l'élément de fixation 8. Cette ouverture oblongue 22 permet d'effectuer une compression osseuse et un rapprochement efficace des fragments d'os 01, 02.

De façon préférentielle, l'élément de fixation 8 comporte en outre une empreinte 23 non débouchante, située de préférence dans la partie sensiblement médiane de sa face externe 12 et au sein de laquelle un outil de serrage, du genre pince, est susceptible de venir prendre appui pour assurer le serrage de l'implant sur le premier métatarsien 1.

En effet, avant et pendant la mise en place des éléments d'ancrage 21, l'utilisation d'une pince peut libérer l'une des mains du chirurgien et lui permettre d'optimiser le positionnement de l'élément de fixation 8. Une telle pince peut par exemple consister en une pince à trois points de serrage, à savoir un première point de serrage au niveau de l'empreinte 23 et deux points de serrage destinés à venir au contact de chacun des fragments d'os 01, 02.

La présente invention peut être utilisée dans une méthode de traitement de pathologies de l'avant pied telles que des fractures ou des déformations du gros orteil, et notamment une méthode d'ostéosynthèse.

En particulier, la présente invention peut être utilisée dans une méthode d'ostéosynthèse de fragments 01, 02 d'un premier métatarsien 1 dans sa partie proximale 1A située vers le tarse, comportant une étape de fixation d'un implant muni d'un élément de fixation 8 sur la surface externe 11 du premier métatarsien, au cours de laquelle on vient, par voie plantaire, positionner l'élément de fixation 8 contre la surface plantaire P proximale du premier métatarsien 1, de telle sorte que ledit élément de fixation 8 s'étende de part et d'autre de la zone de séparation 13 entre les fragments 01, 02.

Plus précisément, on vient positionner directement la face de fixation 10 de l'élément de fixation 8 sur ou contre la surface plantaire P proximale du premier métatarsien 1.

Avantageusement, la méthode comporte, préalablement à l'étape de fixation, une étape de préparation au cours de laquelle on réalise une incision et on ménage un chemin d'accès à partir de la voûte plantaire vers la surface plantaire P du premier métatarsien 1, et ce de manière à permettre l'introduction de l'élément de fixation 8.

Dans le cas d'un traitement d'une déformation du gros orteil, du type « *hallux valgus* », on réalise avantageusement, au cours de l'étape de préparation, une ostéotomie de la partie proximale 1A du premier métatarsien 1, en sectionnant ce dernier de manière à former les fragments d'os 01, 02.

L'élément de fixation 8 présentant une forme anatomique, l'étape de fixation s'effectue directement, sans étape de préformage de l'élément de fixation 8. En effet, grâce à la forme spécifique de l'élément de fixation 8 et notamment à la forme convexe de sa face de fixation 10, aucune étape intermédiaire de pré-formage de l'élément de fixation 8 n'est nécessaire pour lui conférer une forme compatible avec la surface plantaire P. Ainsi, l'élément de fixation 8 présente sa forme fonctionnelle dès sa fabrication, ce qui permet d'éviter au chirurgien d'effectuer une série de pliages laborieux de la plaque 9 de fixation en vue de l'adapter à la forme spécifique de la surface plantaire P.

La méthode chirurgicale décrite constitue ainsi essentiellement une méthode par voie plantaire.

La méthode chirurgicale d'ostéosynthèse va maintenant être décrite en s'appuyant sur les figures 1 à 7.

La méthode chirurgicale est essentiellement une méthode par voie plantaire. Ainsi, lorsqu'une ostéotomie a été réalisée dans la partie proximale 1A du premier métatarsien 1, c'est-à-dire que cet os a été sectionné au moins partiellement en deux fragment d'os 01, 02, on ménage un premier chemin d'accès à partir de la voûte plantaire vers la surface plantaire P proximale du premier métatarsien 1 de manière à permettre l'introduction d'un élément de fixation 8. Cette méthode comprend ensuite une étape dans laquelle on vient positionner directement la face de fixation 10 de l'élément de fixation 8 sur ou contre la surface plantaire P proximale du premier métatarsien 1, de part et d'autre de la zone de séparation 13 entre les fragments d'os 01, 02.

Grâce à la forme spécifique de l'implant de fixation 8 et notamment à la forme convexe de sa face de fixation 10, aucune étape intermédiaire de préformage de l'élément de fixation 8 n'est nécessaire pour lui conférer une forme compatible avec la surface plantaire P. Ainsi, l'élément de fixation 8 présente sa forme fonctionnelle dès sa fabrication, ce qui permet d'éviter au chirurgien d'effectuer une série de pliages laborieux de la plaque 9 de fixation en vue de l'adapter à la forme spécifique de la surface plantaire P.

L'invention permet donc de diminuer considérablement le temps de l'intervention chirurgicale et également de limiter le risque d'erreur opératoire.

Un autre avantage de l'invention est qu'elle permet une pose en aveugle de l'implant de fixation avec un nombre de précautions minimum.

L'invention permet également de réduire de façon significative le risque de jeu après la mise en place de l'implant de fixation sur la surface osseuse du premier métatarsien et donc d'améliorer considérablement la tenue mécanique des fragments d'os ensemble ainsi que la qualité de l'ostéosynthèse.

## Revendications

1. Implant de fixation pour l'ostéosynthèse de fragments (O1, 02) du premier métatarsien (1) dans sa partie proximale (1A), située vers le tarse (5), comprenant au moins un élément de fixation (8), destiné à être fixé, par l'intermédiaire d'une face de fixation (10), sur ou contre la surface externe (11) du premier métatarsien (1), en vue d'assurer la solidarisation des fragments (01, 02) d'os, **caractérisé en ce que** ladite face de fixation (10) comporte au moins une portion de surface anatomique (14) de forme sensiblement conjuguée ou complémentaire à celle de la surface plantaire (P) de la partie proximale (1A) du premier métatarsien (1), la portion de surface anatomique (14) comprenant au moins une première surface dite surface proximale (15), située vers l'extrémité proximale (1A') du premier métatarsien (1), une surface distale (16), située vers l'extrémité de l'élément de fixation (8) opposée à la surface proximale (15), et une surface intermédiaire (17), située entre la surface proximale (15) et la surface distale (16), lesdites surfaces proximale, intermédiaire et distale (15, 17, 16) étant sensiblement inclinées les unes par rapport aux autres.

2. Implant selon la revendication 1 **caractérisé en ce que** la portion de surface anatomique (14) est conformée de manière à venir sensiblement épouser la surface plantaire (P) de la partie proximale (1A) du premier métatarsien (1).

3. Implant selon la revendication 1 ou 2 **caractérisé en ce que** la portion de surface anatomique (14) est sensiblement convexe de manière à s'adapter à la concavité de la surface plantaire (P) de la partie proximale (1A) du premier métatarsien (1).

4. Implant selon l'une des revendications 1 à 3 **caractérisé en ce que** les surfaces proximale, intermédiaire et distale (15, 17, 16) sont sensiblement planes.

5. Implant selon l'une des revendications 1 à 4 **caractérisé en ce que** les surfaces proximale (15) et intermédiaire (17) sont inclinées l'une par rapport à l'autre d'un angle de cintrage proximal (β) compris entre 5° et 30°, préférentiellement de l'ordre de 25°.

6. Implant selon l'une des revendications 1 à 5 **caractérisé en ce que** les surfaces distale et intermédiaire sont inclinées l'une par rapport à l'autre d'un angle de cintrage distal (γ) compris entre 5° et 25°, préférentiellement de l'ordre de 15°.

7. Implant selon l'une des revendications 1 à 6 **caractérisé en ce que** l'élément de fixation (8) s'étend longitudinalement suivant un axe longitudinal (X-X') et les surfaces proximale (15) et intermédiaire (17) se rejoignent au niveau d'un axe de cintrage proximal (Y-Y'), ledit axe de cintrage proximal (Y-Y') étant incliné, par rapport à l'axe longitudinal (X-X'), d'un angle (θ) compris entre 60° et 80°, préférentiellement de l'ordre de 70°.

8. Implant selon l'une des revendications 1 à 7 **caractérisé en ce que** l'élément de fixation (8) s'étend longitudinalement suivant un axe longitudinal et les surfaces distale (16) et intermédiaire (17) se rejoignent au niveau d'un axe de cintrage distal (Z-Z'), ledit axe de cintrage distal (Z-Z') étant incliné, par rapport à l'axe longitudinal, d'un angle (Ω) compris entre 80° et 100°, préférentiellement de l'ordre de 90°.

9. Implant selon l'une des revendications 1 à 8 **caractérisé en ce que** l'élément de fixation (8) comporte un tronçon proximal (19) en forme de spatule, sensiblement plus large que le reste de l'élément de fixation (8).

10. Implant selon l'une des revendications 1 à 9 **caractérisé en ce que** l'élément de fixation (8) comprend au moins deux branches, à savoir une branche proximale (B1) et une branche distale (B2) disposées sensiblement en V.

11. Implant selon la revendication 10 **caractérisé en ce que** l'angle (ϕ) au sommet (S) du V est compris entre 160° et 175°, préférentiellement de l'ordre de 170°.

12. Implant selon l'une des revendications 1 à 11 **caractérisé en ce que** l'élément de fixation (8) est formé par une plaque (9) pourvue d'une face externe (12), opposée à la face de fixation (10), ladite face externe (12) s'étendant de façon sensiblement parallèle à la face de fixation (10).

13. Implant selon la revendication 12 **caractérisé en ce que** ladite face externe (12) est sensiblement lisse.

14. Implant selon l'une des revendications précédentes **caractérisé en ce que** la face de fixation (10) présente un aspect rugueux.

15. Implant selon l'une des revendications précédentes **caractérisé en ce que** l'élément de fixation (8) comporte une pluralité de logements (20) débouchants, répartis sur sa longueur, et adaptés pour recevoir des éléments d'ancrage (21), du genre vis d'ancrage.

16. Implant selon la revendication 15 **caractérisé en ce que** les éléments d'ancrage (21) comportent au moins une vis et une contre-vis, montées en association de manière à assurer le verrouillage en position de l'élément de fixation (8).

17. Implant selon l'une des revendications précédentes **caractérisé en ce que** l'élément de fixation comporte une ouverture sensiblement oblongue (22) débouchante, permettant le rapprochement des fragments d'os (01, 02).

18. Implant selon l'une des revendications précédentes **caractérisé en ce que** l'élément de fixation (8) comporte une empreinte (23), située de préférence dans la partie sensiblement médiane de sa face externe (12), au sein de laquelle un outil de serrage, du genre pince, est susceptible de venir prendre appui pour assurer le serrage de l'implant de fixation (5) sur le premier métatarsien (1).

19. Implant selon l'une des revendications précédentes **caractérisé en ce que** la face externe (12) de l'élément de fixation (8) comporte des bords émoussés.

## Claims

1. A fastener implant for osteosynthesis of fragments (O1, 02) of the first metatarsal bone (1) in its proximal portion (1A) situated towards the tarsal bone (5), the implant comprising at least a fastener element (8) for fastening via a fastener face (10) on or against the outside surface (11) of the first metatarsal bone (1) in order to hold the bone fragments (O1, 02) together, the implant being **characterized in that** said fastener face (10) includes at least one anatomical surface portion (14) of shape that is substantially complementary to the shape of the plantar surface (P) of the proximal portion (1A) of the first metatarsal bone (1), the anatomical surface portion (14) comprising at least a "proximal" surface (15) situated towards the proximal end (1A') of the first metatarsal bone (1), a "distal" surface (16) situated towards the end of the fastener element (8) that is opposite from the proximal surface (15), and an intermediate surface (17) situated between the proximal surface (15) and the distal surface (16), said proximal, intermediate, and distal surfaces (15, 17, 16) being perceptibly inclined relative to one another.

2. An implant according to claim 1, **characterized in that** the anatomical surface portion (14) is shaped in such a manner as to fit substantially snugly against the plantar surface (P) of the proximal portion (1A) of the first metatarsal bone (1).

3. An implant according to claim 1 or claim 2, **characterized in that** the anatomical surface portion (14) is substantially convex so as to match the concave shape of the plantar surface (P) of the proximal portion (1A) of the first metatarsal bone (1).

4. An implant according to any one of claims 1 to 3, **characterized in that** the proximal, intermediate, and distal surfaces (15, 17, 16) are substantially plane.

5. An implant according to any one of claims 1 to 4, **characterized in that** the proximal and intermediate surfaces (15, 17) are inclined relative to each other by a proximal bend angle (β) lying in the range 5° to 30°, and preferably of the order of 25°.

6. An implant according to any one of claims 1 to 5, **characterized in that** the distal and intermediate surfaces are inclined relative to each other by a distal bend angle (γ) lying in the range 5° to 25°, and preferably of the order of 15°.

7. An implant according to any one of claims 1 to 6, **characterized in that** the fastener element (8) extends longitudinally along a longitudinal axis (X-X'), and the proximal and intermediate surfaces (15, 17) are united via a proximal bend axis (Y-Y'), said proximal bend axis (Y-Y') being inclined relative to the longitudinal axis (X-X') at an angle (θ) lying in the range 60° to 80°, and preferably of the order of 70°.

8. An implant according to any one of claims 1 to 7, **characterized in that** the fastener element (8) extends longitudinally along a longitudinal axis, and the distal and intermediate surfaces (16, 17) are united via a distal bend axis (Z-Z'), said distal end axis (Z-Z') being inclined relative to the longitudinal axis at angle (Ω) lying in the range 80° to 100°, and preferably of the order of 90°.

9. An implant according to any one of claims 1 to 8, **characterized in that** the fastener element (8) includes a proximal segment (19) of spatula shape, that is significantly wider than the remainder of the fastener element (8).

10. An implant according to any one of claims 1 to 9, **characterized in that** the fastener element (8) comprises at least two branches, namely a proximal branch (B1) and a distal branch (B2) that are disposed substantially in a V-configuration.

11. An implant according to claim 10, **characterized in that** the angle (ϕ) at the apex (S) of the V-configuration lies in the range 160° to 175°, and is preferably of the order of 170°.

12. An implant according to any one of claims 1 to 11, **characterized in that** the fastener element (8) is formed by a plate (9) provided with an outside face (12) opposite from the fastener face (10), said outside face (12) extending substantially parallel to the fastener face (10).

13. An implant according to claim 12, **characterized in that** said outside face (12) is substantially smooth.

14. An implant according to any preceding claim, **characterized in that** the fastener face (10) presents a rough appearance.

15. An implant according to any preceding claim, **characterized in that** the fastener element (8) includes a plurality of through orifices (20) distributed around its length and adapted to receive anchor elements (21) of the anchor screw type.

16. An implant according to claim 15, **characterized in that** the anchor elements (21) comprise at least one screw and one lock-screw, mounted in association in such a manner as to lock the fastener element (8) in position.

17. An implant according to any preceding claim, **characterized in that** the fastener element includes a substantially oblong through opening (22) enabling the bone fragments (01, 02) to be moved towards each other.

18. An implant according to any preceding claim, **characterized in that** the fastener element (8) includes an indentation (23) preferably situated substantially in the middle portion of its outside face (12) and suitable for receiving a clamping tool of the forceps type so that it can clamp the fastener implant (5) against the first metatarsal bone (1).

19. An implant according to any preceding claim, **characterized in that** the outside face (12) of the fastener element (8) has rounded edges.

## Patentansprüche

1. Fixierungsimplantat zur Osteosynthese der Fragmente (01, 02) des Metatarsus primus (1) in dessen proximalem Teil (1A), der zur Fußwurzel (5) hin angeordnet ist, umfassend mindestens ein Fixierungselement (8), das dazu bestimmt ist, mittels einer Fixierungsfläche (10) auf oder gegen die äußere Oberfläche (11) des Metatarsus primus (1) fixiert zu werden, um die Verbindung der Knochenfragmente (O1, 02) zu gewährleisten, **dadurch gekennzeichnet, dass** die Fixierungsfläche (10) mindestens einen Abschnitt der anatomischen Oberfläche (14) in im Wesentlichen konjugierter oder komplementärer Form zu der Sohlenfläche (P) des proximalen Teils (1A) des Metatarsus primus (1) umfasst, wobei der Abschnitt der anatomischen Oberfläche (14) mindestens eine erste so genannte proximale Oberfläche (15) umfasst, die zum proximalen Endes (1A') des Metatarsus primus (1) hin angeordnet ist, eine distale Oberfläche (16), die zum Fixierungselement (8) hin, gegenüber der proximalen Oberfläche (15) angeordnet ist, und eine intermediäre Oberfläche (17), die zwischen der proximalen Oberfläche (15) und der distalen Oberfläche (16) angeordnet ist, wobei die proximale, die intermediäre und die distale Oberfläche (15, 17, 16) im wesentlichen zueinander geneigt sind.

2. Implantat nach Anspruch 1, **dadurch gekennzeichnet, dass** der Abschnitt der anatomischen Oberfläche (14) ausgerichtet ist, um sich im Wesentlichen an die Sohlenfläche (P) des proximalen Teils (1A) des Metatarsus primus (1) anzuschmiegen.

3. Implantat nach Anspruch 1 oder 2, **dadurch gekennzeichnet, dass** der Abschnitt der anatomischen Oberfläche (14) im Wesentlichen konvex ist, um sich an die Konkavität der Sohlenfläche (P) des proximalen Teils (1A) des Metatarsus primus (1) anzupassen.

4. Implantat nach einem der Ansprüche 1 bis 3, **dadurch gekennzeichnet, dass** die proximale, die intermediäre und die distale Oberfläche (15, 17, 16) im Wesentlichen plan sind.

5. Implantat nach einem der Ansprüche 1 bis 4 **dadurch gekennzeichnet, dass** die proximale (15) und intermediäre (17) Oberfläche zueinander in einem proximalen Biegewinkel (β) im Bereich zwischen 5° und 30°, bevorzugt in der Größenordnung von 25°, geneigt sind.

6. Implantat nach einem der Ansprüche 1 bis 5 **dadurch gekennzeichnet, dass** die distale und intermediäre Oberfläche zueinander in einem distalen Biegewinkel (γ) im Bereich zwischen 5° und 25°, bevorzugt in der Größenordnung von 15°, geneigt sind.

7. Implantat nach einem der Ansprüche 1 bis 6, **dadurch gekennzeichnet, dass** sich das Fixierungselement (8) längs entlang einer Längsachse (X-X') erstreckt und die proximale (15) und intermediäre (17) Oberfläche an einer proximalen Biegeachse (Y-Y') zusammentreffen, wobei die proximale Biegeachse (Y-Y'), bezogen auf die Längsachse (X-X'), in einem Winkel (θ) im Bereich zwischen 60° und 80°, bevorzugt in der Größenordnung von 70° geneigt ist.

8. Implantat nach einem der Ansprüche 1 bis 7, **dadurch gekennzeichnet, dass** sich das Fixierungselement (8) längs entlang einer Längsachse erstreckt und die distale (16) und intermediäre (17) Oberfläche an einer distalen Biegeachse (Z-Z') zusammentreffen, wobei die distale Biegeachse (Z-Z'), bezogen auf die Längsachse, in einem Winkel (Ω) in Bereich zwischen 80° und 100°, bevorzugt in der Größenordnung von 90° geneigt ist.

9. Implantat nach einem der Ansprüche 1 bis 8, **dadurch gekennzeichnet, dass** das Fixierungselement (8) ein proximales Teilstück (19) in Form eines Spatels umfasst, der im Wesentlichen breiter ist als der Rest des Fixierungselements (8).

10. Implantat nach einem der Ansprüche 1 bis 9, **dadurch gekennzeichnet, dass** das Fixierungselement (8) mindestens zwei Schenkel, und zwar einen proximalen Schenkel (B1) und einen distalen Schenkel (B2) umfasst, die im Wesentlichen in V-Form angeordnet sind.

11. Implantat nach Anspruch 10, **dadurch gekennzeichnet, dass** der Winkel (ϕ) an der Spitze (S) des V im Bereich zwischen 160° und 175°, bevorzugt in der Größenordnung von 170° liegt.

12. Implantat nach einem der Ansprüche 1 bis 11, **dadurch gekennzeichnet, dass** das Fixierungselement (8) durch eine Platte (9) gebildet wird, die mit einer äußeren Fläche (12) gegenüber der Fixierungsfläche (10) versehen ist, wobei sich die äußere Fläche (12) im Wesentlichen parallel zur Fixierungsfläche (10) erstreckt.

13. Implantat nach Anspruch 12, **dadurch gekennzeichnet, dass** die äußere Fläche (12) im Wesentlichen glatt ist.

14. Implantat nach einem der vorhergehenden Ansprüche, **dadurch gekennzeichnet, dass** die Fixierungsfläche (10) ein raues Aussehen aufweist.

15. Implantat nach einem der vorhergehenden Ansprüche, **dadurch gekennzeichnet, dass** das Fixierungselement (8) eine Vielzahl von Durchgangssitzen (20) umfasst, die über seine Länge verteilt und angepasst sind, um Verankerungselemente (21) vom Typ Verankerungsschraube aufzunehmen.

16. Implantat nach Anspruch 15, **dadurch gekennzeichnet, dass** die Verankerungselemente (21) mindestens eine Schraube und eine Konterschraube umfassen, die in Assoziation befestigt sind, um die örtliche Blockierung des Fixierungselements (8) zu gewährleisten.

17. Implantat nach einem der vorhergehenden Ansprüche, **dadurch gekennzeichnet, dass** das Fixierungselement eine im Wesentlichen längliche (22) Durchgangsöffnung umfasst, die das Zusammenschieben der Knochenfragmente (O1, 02) ermöglicht.

18. Implantat nach einem der vorhergehenden Ansprüche, **dadurch gekennzeichnet, dass** das Fixierungselement (8) eine Vertiefung (23) umfasst, die bevorzugt im im Wesentlichen mittleren Teil seiner äußeren Fläche (12) liegt, in der ein Spannwerkzeug, vom Typ Klemme, befähigt ist, sich abzustützen, um das Spannen des Fixierungsimplantats (5) auf den Metatarsus primus (1) zu gewährleisten.

19. Implantat nach einem der vorhergehenden Ansprüche, **dadurch gekennzeichnet, dass** die äußere Fläche (12) des Fixierungselements (8) abgestumpfte Ränder umfasst.
